Europäisches Patentamt

(19) European Patent Office

Office européen des brevets

(11) Publication number: 0 384 541
A1

# (12) EUROPEAN PATENT APPLICATION

(21) Application number: 90200416.7

(51) Int. Cl.5: C12N 11/04, A23L 3/34

(22) Date of filing: 21.02.90

(30) Priority: 24.02.89 EP 89200472

(43) Date of publication of application:
29.08.90 Bulletin 90/35

(84) Designated Contracting States:
AT BE CH DE DK ES FR GB GR IT LI LU NL SE

(71) Applicant: GIST-BROCADES N.V.
Wateringseweg 1 P.O. Box 1
NL-2600 MA Delft(NL)

(72) Inventor: Edens, Luppo
Hoflaan 118
NL-2062 JL Rotterdam(NL)

(74) Representative: Matulewicz, Emil Rudolf
Antonius, Dr. et al
Gist-Brocades NV Patents and Trademarks
Department Wateringseweg 1 P.O. Box 1
NL-2600 MA Delft(NL)

(54) Long active food grade oxygen scavenger that can withstand pasteurization.

(57) A process is disclosed for immobilising a dried microorganism in solid material which comprises forming a suspension by bringing together, in a substantially anhydrous organic solvent, the dried viable microorganism and solid material, which dissolves in the organic solvent, coating a surface with the suspension, and removing the solvent. One application of the process in the deoxygenation of containers of food and drink that have to be pasteurised, wherein dried yeast is immobilised on the inner surface, stopper or closure of a container such that it retains, during pasteurisation, its oxygen consuming capacity.
Another application of the invention is the use in the food industry of foils, wherein yeast is immobilised.

EP 0 384 541 A1

## LONG ACTIVE FOOD GRADE OXYGEN SCAVENGER THAT CAN WITHSTAND PASTEURIZATION

The present invention relates to a coating process which can be applied to obtain immobilized dried viable microorganisms on a solid material.

Food or beverage deterioration by oxygen occurs during storage of many consumable products. The presence of oxygen encourages microbial activity and various oxidation reactions. In this way, oxygen is known to adversely influence the microbial and flavor stability of foodstuffs like cheese and meat products, juices, ketchup and more particularly oil and fat containing products like margarines and nuts, and beverages like beer and wine.

EP-A-0305005, filed on August 22, 1988 describes the incorporation of yeast in a coating in such a way that the yeast survives pasteurization treatment and is not in direct contact with the product being pasteurised. The yeast is immobilised in a solid material which allows water to penetrate to the yeast only slowly. When the yeast is still in a dry immobilised condition, the container with product may be pasteurized without losing all the activity of the yeast. The yeast which is a 'natural ingredient' will consume oxygen thereby producing carbon dioxide and water.

Microorganisms, and yeasts in particular, are known to withstand temperatures well above 65°C for a limited period of time as long as they contain only a few percent of water. However, in a wetted state, they cannot withstand temperatures higher than 55-60°C for more than a few minutes without losing all their metabolic capacity. Therefore, to survive a pasteurization procedure, the microorganism has to be substantially dry. After the pasteurization treatment, in contrast, wetting of the yeast is essential for its activation.

Coatings containing dried yeast may be applied as food-grade oxygen scavengers for water containing products with the aim of maintaining the quality of such products during storage. Examples of such products, all of which have to include a minimal amount of water, are beverages or oils, fats, oil and/or fat containing products.

EP-A-0305005 describes the removal of oxygen from pasteurized and non-pasteurized containers using yeasts which are immobilized in waxes or polymers. The process comprises melting the waxes or polymers and then adding the dried yeast to form a slurry, which is applied as a coating to the inside surface of, for example, a crown cork. As a result of the immobilization procedure, the yeasts can survive pasteurization and yet growth in the product is prevented because viable yeasts are not liberated into the product.

A major disadvantage of the above process is that only a limited number of waxes or polymers can be used. They must not only be able to withstand pasteurisation temperatures, but must also melt at a temperature which is not so high that the dry yeast would be killed when added to the molten wax or polymer. Moreover, the wax or polymer has to be acceptable for use in direct contact with beverages or food products for human consumption. Finally, after being applied to a surface, this wax or polymer, has to remain fixed on the surface for a long period of time. In particular, the attachment of wax to polymer surfaces can give rise to problems.

Although this oxygen removal method has been shown to work when used for beer bottles, the limitations and shortcomings described above restrict technical and commercial utility of the method.

Coatings comprising synthetic macromolecular compounds (synthetic polymers) have found wide acceptance in the protective coating field. When properly formulated, low levels of odour, taste and water vapour transmission are characteristic of these polymers. PVC, polystyrene and polycarbonate are examples of synthetic polymer, frequently used for such coatings. However, in general these polymers cannot be applied in hot-melting techniques. The polymers may instead be dissolved in a suitable solvent and applied to the surface to be coated by one of the conventional liquid coating techniques. After evaporation of the solvent a dry, polymer-coated surface is obtained. Similarly such synthetic polymers in the form of foils are used in the food and packaging industry to protect a product against external influences.

From the literature it is known that active yeast can be extracted with organic solvents in order to remove from it compounds like hydrocarbon(s) and lipid(s). During this extraction process the yeast cells are killed. Therefore the use of organic solvents in any process, requiring a metabolically active yeast would be expected to be unacceptable. Further, it is well known in the medical field that alcohol can be used as disinfectant in order to make a surface effectively free of microbial infection.

It has now surprisingly been found that by using dried microorganisms, such as dried yeast, in combination with solvents which are substantially anhydrous, the microorganisms can survive a solvent treatment process to the extent of at least 10%, usually 30% and more typically 50% of the microorganisms initially present. The present invention is based on this finding. The dried microorganisms have preferably more than 92 wt%, more preferably 94 to 98 wt% dry matter.

Accordingly the present invention provides a process for the preparation of an immobilized dried microorganism which comprises forming a suspension by bringing together in a substantially anhydrous organic solvent, a dried microorganism and a solid material, which dissolves in the organic solvent, coating a surface with the suspension and removing the solvent to form an immobilized dried microorganism in the solid material. The microorganism is, for example, yeast and the solid material is, for example, a polymer. These solvent-containing coatings can be applied by conventional fluid techniques such as spraying, dipping, brushing and roll coating.

The present invention provides, in one embodiment, a process for immobilizing dry yeasts in a food-grade polymer, which polymer allows water to penetrate only very slowly. As a result, most of the yeast will remain essentially dry during pasteurization but will become wetted and hence active during prolonged exposure to a water-saturated atmosphere as exists in the head space of a closed container filled with the water-containing product.

The invention may be applied to immobilize all kinds of yeasts so that it becomes possible to carry out biological conversions in which the yeast is easily separated from substrates and products. Outgrowth of the yeast is prevented by the solid material. Outgrowth of the yeast can also be prevented by providing the dried immobilised yeast in the solid material with a further protective coating which prevents outgrowth.

Although the invention will be described in some detail by way of illustration and example for purposes of clarity and understanding on the basis of the use of a yeast, preferably a Saccharomyces cerevisiae, to remove oxygen from containers for food and drink, it will be readily apparent to those of ordinary skill in the art that the process of this invention enables yeasts to be used in other microbiological processes where immobilisation on a surface is required.

The process of the present invention provides a coating (solid material) for a surface in which a dried microorganism is incorporated, thereby immobilizing and protecting said microorganism. After the coating process at least a substantial part of the immobilized microorganism is viable and able to carry out essential parts of its normal microbiological reactions upon wetting the immobilized microorganism with water or water vapour.

The process of the present invention for immobilizing a dried microorganism on a surface is typically carried out by dissolving the solid material in the organic solvent and adding the dried micoorganism thereto. The solid material is typically a polymer. Subsequently a surface is coated with the suspension thus formed using conventional fluid techniques, whereafter the solvent is removed from the suspension. In this way it is possible to produce foils containing the immobilized microorganisms. Foils are widely applied nowadays in the food industry.

As described above, different types of coating materials can be used depending on the desired properties of the coating in connection with the immobilized microorganism. The organic solvents used are substantially anhydrous. "Substantially anhydrous" means free of water, but allows for the presence of traces of water. The choice of the solvent is determined by the coating material, the surface to be coated, the solvent and recovery facilities available. For example, vinyl chloride polymers and copolymers are normally soluble in ketones; they have varying degrees of solubility in toluene or xylene. Therefore by solvent is meant a volatile liquid of sufficient dissolving potency to completely dissolve the coating material. Important additional considerations for these solvents are toxicity, evaporation rate, flash point, distillation range, density, specific gravity, odour, colour, acidity, content of non volatile matter and purity. Suitable solvents are for example methyl ethyl ketone, methyl isobutyl ketone, cyclohexane, methylene chloride.

The solid material used for immobilization of the yeast should not only dissolve in the solvent but should also be acceptable for use in direct contact with beverages or food-products if intended for human consumption, i.e. the material should then be "food-grade". If the coating is used for other purposes, the skilled person will understand that other demands are placed on the coating. If immobilized yeast has to withstand pasteurization and is used to remove oxygen, the material should have acceptable properties with respect to permeability for oxygen, carbon dioxide and water. It will be appreciated that in other processes other properties may be preferred depending on the compounds involved in the process. Suitable solid materials for the process of yeast immobilization include synthetic polymers, for example, PVC, polystyrene and polycarbonate. Optionally these polymers may be blended with improvers. By improver is meant an agent for improving the attachment of the immobilizing material to the carrier surface.

It will be appreciated by a person skilled in the art that an immobilizing material has to be used which adheres satisfactorily to the surface in question. Often surface preparation is essential before the coating is fixed.

The immobilizing materials may advantageously be applied as a thin layer or film containing the microorganism to packaging foils or other packaging materials typically used for food or drink or, alternatively, applied to the inner surface of containers or to the surface of stoppers or closures for containers such as crown corks, "snap" caps or tops. Contact with the packed product may occur but preferably the immobilised microorganism is in contact only with the gases and vapours above the product.

After evaporation of the organic solvent the synthetic polymers should preferably have a limited permeability to water vapour and preferably a high transmission rate for gases like oxygen and carbon dioxide in those cases where the microorganisms has to withstand pasteurization and is used to remove oxygen. In cases where the microorganism will not become pasteurised, a high transmission rate for gases suffice. To withstand pasteurization the permeability of the polymer to water vapour should preferably be less than 100 units $PH_2O$ whereas the transmission rate for oxygen should be greater than 0.01 units Pox. More preferably the permeability to water vapour is less than 10 units $PH_2O$ and the permeability to oxygen greater than 0.1 units Pox. The permeability unit P used is defined as the "barrer", the standard unit of P adopted by ASTM. More precisely the permeability of $PH_2O$ is defined as

$$\frac{(cm^3 \text{ at STP})(mm \text{ thickness} \times 10^8)}{(cm^2 \text{ area})(s)(cmHg)}$$

and the permeability unit Pox as

$$\frac{(cm^3 \text{ at STP})(mm \text{ thickness} \times 10^{10})}{(cm^2 \text{ area})(s)(cmHg)}$$

at 25°C and are described in "Polymer Permeability" by J. Comijn (Elsevier Applied Science Publishers; 1985) pages 61-63.

In an embodiment of the invention dried yeasts are mixed with a dissolved immobilising material, such as a synthetic polymer, after which the mixture is applied to the inside of a cork or stopper with which a container is to be closed. Advantageously about 1-50 w/w% of immobilizing material is added to the solvent and later on about 0.1-30 w/w% of yeast. Nowadays bottles are frequently closed using crown corks provided with a polymer coating (e.g. polyvinylchloride = PVC or polyethylene = PE) on the inside, see e.g. Chemical and Engineering News, February 8 (1965), pp. 43-44, UK patent application GB 1,211,780 and Japanese patent applications J48032086 and J50112181. The mixture of microorganisms and immobilising material may be applied on top of this coating so that a final layer having a thickness of between 5 $\mu$m-2 mm is attached to the inside of the crown cork. Such layers should contain between 0.01-160 milligrams, preferably between 1-40 milligrams, of dry yeast per square centimetre of coating.

Oxygen scavenging capacity is determined by the amount of reserve nutrients within the dry yeast. In the fermentation procedure these reserve nutrients can be maximized. The rate by which these reserves are utilized depends upon the rate by which oxygen is supplied e.g. the more oxygen is supplied the faster the yeasts reserves wear out. By adjusting the quantity of yeast, very low oxygen levels can be obtained.

It will be appreciated that the immobilized yeast is preferably not present to any significant extent on the sealing line itself. For example, in the case of a bottle with a polymer coated crown cork, the yeast should not be present between the lip of the bottle and the liner of the crown cork.

Before use on e.g. a bottle, crown corks provided with the protected yeast are preferably stored in such a way that air humidity is excluded, e.g. in bags containing suitable drying agents. Foils are advantageously stored on a roll. By prolonged exposure to humid air, water slowly penetrates the synthetic polymer coating so that the water content of the dry microorganism increases. As a result the microorganism will not survive pasteurization.

Nevertheless part of the immobilized yeast may become wetted during storage or application. This part of the yeast may be killed during pasteurization. However, it has been found that this affects only the outer surface layers. The killing of the outer layer of the yeast even has the advantage that it becomes more difficult for the yeast to become in direct contact with the water containing product.

Normally in the time between pasteurization and consumption of the water containing product, the yeast has removed oxygen from the container. Even when the container is cooled, for example by the consumer, the yeast will remain sufficiently active to prevent the oxygen level in the container increasing.

The yeast which can be used according to the present invention is, for example, any yeast belonging to the genus Saccharomyces, Kluyveromyces or Schizosaccharomyces.

The following Examples are given to illustrate the invention.

Example 1

Samples of 1 gram active dry yeast powder (96% dry matter, Fermipan (TM), Gist Brocades) and samples of 1 gram compressed wet yeast (33% dry matter, fresh baker's yeast) were mixed with 0.5 ml of water-free organic solvents. After incubation of the mixtures at 20°C for 1 hour and 24 hours, respectively, the temperature of the samples was increased till 37°C after which solvents were removed by vacuum evaporation. The solvent-free samples were suspended in 100 ml water of 30°C containing 0.85% NaCl. Serial dilutions in the same medium and plating of 0.1 ml samples on malt extract agar containing Petri dishes followed by incubation at 30°C for 2 days allowed estimation of the numbers of viable yeasts present in each yeast-solvent sample.

The results, representing the average value obtained in 3 experiments, are given in Table 1. In all cases the figures represent the total viable cells per gram dry matter of yeast.

The data obtained clearly demonstrate that the solvent treatment of "wet" cells decreases their viability by 3 to 4 orders of magnitude whereas a similar treatment of "dry" cells has a limited effect only. Furthermore Table 1 shows that a broad variety of organic solvents did not influence the long term viability of dried yeasts. Therefore the solvent can often be selected by the processing demands.

Table 1

|  | Number of viable yeast cells (yeast of 33% dry matter) after solvent incubation for: | | Number of viable yeast cells (yeast of 96% dry matter) after solvent incubation for: | |
| --- | --- | --- | --- | --- |
| Solvent type: | 1 hour | 24 hours | 1 hour | 24 hours |
| Water (reference) | $8.10^{10}$ | $6.10^{10}$ | $1.10^{11}$ | $1.10^{11}$ |
| Ethanol (100%) | $< 3.10^6$ | $6.10^3$ | $1.10^{11}$ | $6.10^{10}$ |
| Acetone | $< 3.10^6$ | $< 3.10^3$ | $1.10^{10}$ | $1.10^{10}$ |
| Methylene chloride | $< 3.10^6$ | $< 3.10^3$ | $1.10^{11}$ | $7.10^{10}$ |
| MEK* | $3.10^6$ | $3.10^6$ | $1.10^{11}$ | $5.10^{10}$ |
| MIBK** | $< 3.10^6$ | $< 3.10^6$ | $4.10^{10}$ | $1.10^{10}$ |
| Cyclohexane | $3.10^7$ | $3.10^7$ | $3.10^{10}$ | $1.10^{10}$ |
| Toluene | $< 3.10^6$ | $< 3.10^3$ | $3.10^9$ | $1.10^9$ |

* MEK = methyl ethyl ketone (2-butanone)

** MIBK = methyl isobutyl ketone (4-methyl-2-pentanone)

Example 2

The following synthetic polymers were used in this example:
1. polycarbonate (Makrolon 2808, Bayer)
2. polystyrene (Vestyron 325-31, Hüls AG)
3. polyethylene co-vinylacetate (Elvax 40-W, Dupont)
4. PVC (Vestolit B 7021, Hüls AG).

Samples of 45 grams of a polymer were dissolved in 400 ml water-free $CH_2Cl_2$. The solution was reduced in volume by evaporation to a volume of 200 ml and 5 grams of dry yeast (Saccharomyces cerevisiae, 96 wt% dry matter) was added and stirred until a homogenous suspension was obtained. The suspension was quickly poured out in a mould of 20 x 20 cm and 1 mm height. The mould was supported by filtration paper laying on a glass plate. The total amount of the suspension was poured into the mould in three successive portions to promote the evaporation of $CH_2Cl_2$. In the case of Vestolit it was essential to add dioctylphtalate to improve film formation. Rather than using 45 grams of synthetic polymer, in the case of Vestolit B 7021 30 grams of polymer and 15 grams of dioctylphtalate was dissolved. After drying, the mould was removed and the plastic layer containing the embedded yeast remained fixed to the filter paper.

The yeast-synthetic polymer preparations were stored in a dry atmosphere in the presence of silicagel until use.

The oxygen consumption rates of these yeast-coating preparations were determined in regular 200 ml bottles the volume of which was reduced by filling the bottle with 90 grams of inert wax (Dicera 8582). Actual remaining volume in all bottles was 124 ml. Subsequently 1 ml of water was added to all bottles (to create a water saturated atmosphere in the sealed bottle) as well as 20 cm² (approximately 2 x 10 cm pieces) of aforementioned yeast-coating preparations. Calculated on the basis of yeast introduced, each bottle contained 0.23 grams of dry yeast. After having introduced the various yeast-coating preparations, the bottles were evacuated and filled with a gas mixture consisting of 95 v/v% $N_2$ and 5 v/v% $O_2$. Sealing of the bottles with a crown cork took place in the same nitrogen-oxygen gas atmosphere. Reference bottles containing synthetic polymer preparations of the same dimensions but without yeast were subjected to the same procedure.

To test the heat stability of the various yeast-coating preparations, half of the bottles containing the various yeast-coating preparations were subjected to a heat treatment of 20 minutes at 70° C. The other half of the bottles (and 2 bottles without yeast) were not heated at all. Immediately after their heat treatment, the bottles were cooled down and stored in the dark at 20° C together with the unheated bottles. Gas samples (1 bottle = 1 sample) were taken after various incubation periods. The oxygen content of each sample was determined using the head-space method and analysed using a gaschromatographic technique (katharometer detection using hydrogen as the carrier gas).

The oxygen concentration in reference bottles without yeasts remained stable around 5% over a period of 16 days at least. The oxygen concentration in unheated bottles containing the various yeast-coating preparations decreased to less than 0.1 v/v% within an average period of 6 days (range 2-9 days). The bottles which were heated for 20 minutes at 70° C showed oxygen concentrations of less than 0.1 v/v% within an average period of 10 days (range 2-16 days).

These data clearly demonstrate that the oxygen uptake capacity of dried yeast can withstand an immobilisation by immersion in dissolved synthetic polymers. The data demonstrate furthermore that coated yeast preparations are able to survive heat treatments at 70° C for 20 minutes at least.

## Claims

1. A process for the preparation of an immobilized dried microorganism which comprises:
forming a suspension by bringing together, in a substantially anhydrous organic solvent, a dried microorganism and a solid material, which dissolves in the organic solvent;
coating a surface with the suspension; and
removing the solvent to form an immobilized dried microorganism in the solid material.

2. A process according to claim 1, wherein the dried microorganism is a yeast, for example Saccharomyces cerevisiae.

3. A process according to claim 1 or 2, wherein the dried microorganism comprises at least 92 wt%, for example 94 to 98 wt% dry matter.

4. A process according to any one of claims 1 to 3, which comprises dissolving the solid material in the organic solvent and adding the dried microorganism thereto.

5. A process according to any one of claims 1 to 4 wherein the solid material is a polymer, optionally a food-grade polymer.

6. A process according to claim 5 wherein the polymer has a permeability to water vapour of 100 units $PH_2O$ or less and a transmission rate for oxygen of at least 0.01 units Pox.

7. A process according to any one of claims 1 to 6 which comprises coating the surface to a thickness of 5 $\mu$m to 2 mm, preferably of 10 to 500 $\mu$m.

8. A process according to claim 7 which comprises the production of a foil.

9. A process according to any one of claims 1 to 7 wherein the surface is the inner surface of a container or the surface of a stopper or other closure for a container.

10. A process according to claim 9 which comprises the step of subjecting the container to pasteurisation once the inner surface has been coated or the coated stopper or closure has been inserted into the container.

11. A suspension comprising a dried viable microorganism, an organic solvent and a polymer.

12. A dried viable microorganism immobilized in a solid material obtainable by a process according to claim 1.

13. A dried viable microorganism immobilized in a foil obtainable by a process according to claim 1.

14. A dried viable microorganism according to claim 12 or 13, wherein said microorganism is a yeast, preferably Saccharomyces cerevisiae.

15. A dried viable yeast according to claim 14, wherein the solid material allows very slow penetration of water to the yeast and allows rapid penetration of oxygen to the yeast.

16. A dried viable yeast according to claim 14 or 15, wherein the solid material is acceptable for use in direct contact with beverages or food products for human consumption.

17. A dried viable yeast according to claim 14, wherein part of the yeast cells may survive treatment at elevated temperature.

18. A stopper or closure for a container having at least a part of its surface coated with a dried viable microorganism, preferably a yeast immobilized in a solid material, obtainable by a process according to claim 1.

19. A stopper or closure according to claim 18 which is a crown cork having the coating on a surface that is intended to be on the inside of the closed container.

20. A closed container wherein at least a part of the inner surface of the container is coated with a dried viable microorganism, preferably a yeast immobilized in a solid material, obtainable by a process according to claim 1.

21. A process for removing oxygen from a closed container wherein a quantity of dried viable yeast according to claim 14 is introduced into the container in such a way that the viable yeast is not allowed to propagate substantially.

22. A process according to claim 21 wherein the container and its contents is subjected to a pasteurization treatment when the viable yeast is still in substantial dry condition.

23. Use of a suspension according to claim 11 in a coating process.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | FR-A-1 306 640  (F. LEUSCHNER) * Page 2, column 1; page 2, column 2, paragraphs 1,2,4; examples 1,4; summary * | 1-23 | C 12 N  11/04 A 23 L   3/34 |
| A | US-A-4 510 162  (J.W. NEZAT) * Claims 1,2,16 * | 1,2,5,6 ,14,20 | |
| X | EP-A-0 086 179  (CONSIGLIO NAZIONALE DELLE RICERCHE) * Claims * | 1,3,4, 17 | |
| A | GB-A-1 224 947  (SNAM PROGETTI S.p.A.) * Claims * | 1 | |
| A | GB-A-2 046 271  (MITSUBISHI RAYON CO. LTD) * Claims; page 2, lines 10-45,58-65 * | 1,2,4, 11,12 | |
| A | DE-C- 839 245  (E. KANZ) * Figures; page 3, lines 5-16 * | 1 | |

TECHNICAL FIELDS
SEARCHED (Int. Cl.5)

A 23 L
C 12 N
C 12 H

The present search report has been drawn up for all claims

| Place of search | Date of completion of the search | Examiner |
|---|---|---|
| THE HAGUE | 09-04-1990 | COUCKE A.O.M. |